# NEUE EUROPÄISCHE PATENTSCHRIFT

(11) **EP 0 945 124 B2**
(45) Veröffentlichungstag und Bekanntmachung des Hinweises auf die Entscheidung über den Einspruch: **02.06.2010**
(45) Hinweis auf die Patenterteilung: 02.05.2007
(21) Anmeldenummer: 99103602.1
(22) Anmeldetag: 24.02.1999
(51) Int. Cl.: A61K 8/41, A61K 8/60, A61Q 5/12

(54) **Haarbehandlungsmittel**
Hair treatment composition
Agent pour le soin de la chevelure

(30) Priorität: 09.03.1998 DE 19810122
(43) Veröffentlichungstag der Anmeldung: 29.09.1999
(73) Patentinhaber: KPSS-Kao Professional Salon Services GmbH, 64297 Darmstadt (DE)
(72) Erfinder: Fath, Bettina, 69469 Weinheim (DE); Bistram, Vera, 64683 Einhausen (DE)

(56) Entgegenhaltungen:
- WO-A-94/07978
- DE-C- 19 732 015

## Beschreibung

Die vorliegende Erfindung betrifft ein Haarbehandlungsmittel mit verbesserten konditionierenden Eigenschaften, insbesondere verbesserter Naß- und Trockenkämmbarkeit, vollem und gleichzeitig lockerem Griff sowie gutem Halt und lebhaftem Glanz, das nach dem Auftragen auf dem Haar verbleiben oder wieder ausgewaschen werden kann.

Es ist bereits seit längerem bekannt, Haarbehandlungsmitteln, insbesondere Haarnachbehandlungsmitteln im Sinne der Definition in der Monographie von K. Schrader, Grundlagen und Rezepturen der Kosmetika, 2. Auflage (1989, Hüthig Buch Verlag), S. 722-724, haarkonditionierende Wirkstoffe wie Polymere und/oder quaternäre Ammoniumverbindungen mit ein oder zwei langkettigen Alkylresten zuzusetzen.

Eine Optimierung der Wirksamkeit entsprechender Haarnachbehandlungsmittel läßt sich durch den kompletten oder zumindest teilweisen Einsatz dieser quaternären Ammoniumverbindungen durch Verbindungen, die neben langkettigen Alkylketten und quaternären Ammoniumgruppen noch mindestens eine Estergruppe im Molekül aufweisen, sogenannte "Esterquats", erzielen. Entsprechende, diese Esterquats enthaltende Haarpflegemittel sind beispielhaft in der WO 94/06899 A, der EP 0 614 349 B 1 und der EP 0 680 314 B1 beschrieben. In WO 94/07978 wird Glucose als Dispergierhilfsmittel in trockenen Detergenzgemischen eingesetzt.

Es wurde nunmehr gefunden, daß sich die haarkonditionierende Wikrsamkeit von Esterquats enthaltenden Haarbehandlungsmitteln signifikant verbessern läßt, wenn ein solches Mittel auf wäßriger Grundlage ein Gemisch aus
a) 0,1 bis 20, insbesondere 0,5 bis 10 Gew.-%, mindestens einer Verbindung der allgemeinen Formel I 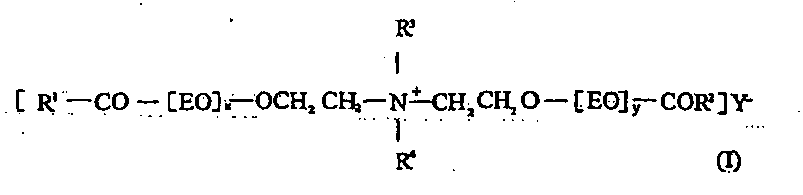 in der R¹ und R² jeweils für eine gegebenenfalls hydroxysubstituierte C₈-C₂₂- Alkyl- oder Alkenylgruppe, R³ und R⁴ für eine C₁-C₃-Alkylgruppe oder eine Gruppe -CH₂-CH₂-O-[EO]_{z}-H, sowie x,y und z für 0 bis 5, und Y⁻ für ein Anion stehen und
b) 0,1 bis 20, vorzugsweise 0,25 bis 10 Gew.-% mindestens eines Mono- und/oder Oligosaccharids enthält.
jeweils berechnet auf die Gesamtzusammensetzung des Mittels, wobei das Mittel ein Mono- und/oder Oligosaccharid ausgewählt aus Saccharose, Fructose, Lactose, Maltose, Galactose, Cellobiose, Fucose, Mannose, Erythrose und Rhamnose enthält.

Bevorzugte Reste R¹ und R² in der Verbindung der Formel I sind C₁₂-C₁₈-Alkyl- und Oleylreste, der Rest R³ eine Methylgruppe und der Rest R⁴ eine Hydroxyethylgruppe, x,y und z sind vorzugsweise 0 oder 1; Y⁻ ist vorzugsweise ein Methosulfat-, Chlorid- oder Phosphatanion.

Diese Verbindungen sind, wie oben bemerkt, an sich bekannt und beispielsweise unter den Markenbezeichnungen "Schercoquat^{R}", Dehyquart^{R}F30" und "Tetranyl^{R}" im Handel.

Als Mono-und/oder Oligosaccharide in den erfindungsgemäßen Shampoos werden vorzugsweise Saccharose, Fructose, Lactose, Maltose, Galactose, Cellobiose, Fucose, Mannose, Erythrose, Rhamnose, etc. eingesetzt.

Es ist zweckmäßig, diese Zucker als Gemische, beispielsweise als Bestandteile von Naturstoffen, zu verwenden.

Ein bevorzugter, Mono- und Disaccharide enthaltender Naturstoff ist beispielsweise Honig. Auch Stärkehydrolysate sind geeignet.

Der Anteil in der Gesamtzusammensetzung bezieht sich dabei jeweils auf den Mono- bzw. Oligosaccharidanteil.

Besonders bevorzugt sind dabei 0,5 bis 5 Gew.-% der Gesamtzusammensetzung.

Die erfindungsgemäßen Zusammensetzungen enthalten vorzugsweise noch mindestens ein kationisches Tensid, insbesondere in einer Menge von 0,1 bis 7,5, vorzugsweise 0,25 bis 5, besonders bevorzugt 0,5 bis 2,5 Gew.-%, der Gesamtzusammensetzung.

Geeignete langkettige quaternäre Ammoniumverbindungen, die als kationische Tenside allein oder im Gemisch miteinander eingesetzt werden können, sind insbesondere Cetyltrimethylammoniumchlorid, Dimethyldicetylammoniumchlorid, Trimethylcetylammoniumbromid, Stearyltrimethylammoniumchlorid, Dimethylstearylbenzylammoniumchlorid, Benzyltetradecyldimethylammoniumchlorid, Dimethyldihydriertes-Talgammoniumchlorid, Laurylpyridiniumchlorid, Lauryldimethylbenzylammoniumchlorid, Lauryltrimethylammoniumchlorid, Tris(oligooxyethyl)alkylammoniumphosphat, Cetylpyridiniumchlorid, Behenyltrimoniumchlorid, etc.

Gut geeignet sind auch die in der EP-A 472 107 geoffenbarten quaternären Ammoniumsalze. Im Prinzip sind alle quaternären Ammoniumverbindungen, wie sie im "CTFA International Cosmetic Ingredient Dictionary", Fourth Ed. (1991), unter dem Trivialnamen "Quaternium" aufgeführt sind.

Die Zusammensetzung kann natürlich zusätzlich die in solchen Konditionierungsmitteln üblichen Bestandteile enthalten; es wird, zur Vermeidung von Wiederholungen, auf K.Schrader, S.722 - 771, verwiesen.

Auch nichtionische Tenside können, insbesondere im Gemisch mit kationaktiven Tensiden, Verwendung finden, beispielsweise Aminoxide in einer Menge von etwa 0,25 bis etwa 5, vorzugsweise etwa 0,5 bis etwa 3,5 Gew.-%, berechnet auf die Gesamtzusammensetzung des Mittels.

Solche Aminoxide gehören seit langem zum Stand der Technik, beispielsweise C₁₂-C₁₈-Alkyldimethylaminoxide wie Lauryldimethylaminoxid, C₁₂-C₁₈-Alkylamidopropyl oder - ethylaminoxide, C₁₂-C₁₈-Alkyldi(hydroxyethyl)- oder -(hydroxypropyl)aminoxide, oder auch Aminoxide mit Ethylenoxid- und /oder Propylenoxidgruppen in der Alkylkette.

Geeignete Tenside sind weiterhin die bekannten C₈-C₁₈-Alkylpolyglucoside, insbesondere mit einem Polykondensationsgrad von 1,2 bis 3, insbesondere solche mit der allgemeinen Formel

R-O-(R¹O)ₙ-Zₓ ,

worin R eine Alkylgruppe mit 8 bis 18 Kohlenstoffatomen, R¹ eine Ethylen- oder Propylengruppe, Z einen Saccharidrest mit 5 bis 6 Kohlenstoffatomen, n eine Zahl von 0 bis 10 und x eine Zahl zwischen 1 und 2,5 bedeuten.

Andere nichtionische Tenside sind beispielsweise die verschiedenen Sorbitanester, wie Polyethylenglykolsorbitanstearinsäureester, Fettsäurepolyglykolester oder auch Mischkondensate aus Ethylenoxid und Propylenoxid, wie sie beispielsweise unter der Handelsbezeichnung "Pluronics" im Verkehr sind.

Auch amphotere bzw. zwitterionische Tenside können mitverwendet werden, beispielsweise Betaine, Sulfobetaine und/oder Alkylaminocarbonsäuren wie Alkylaminoglycinate, Alkylaminopropionate, Alkylglycinate, etc. eingesetzt werden. Besonders geeignet sind Alkylamidobetaine der allgemeinen Formel 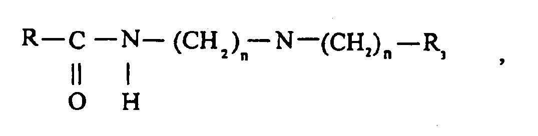 wobei R eine C₈-C₁₈-Alkylgruppe, z. B. einen Cocoalkylrest; R₁ und R₂ einen niederen C₁-C₄-Alkyl- oder -Hydroxyalkylrest, insbesondere eine Methyl-, Ethyl- und/oder Hydroxyethylgruppe; R₃ eine COO⁻ -oder -SO₃⁻ -Gruppe; und n 1 bis 3 bedeuten.

Auch Betaine der allgemeinen Formel 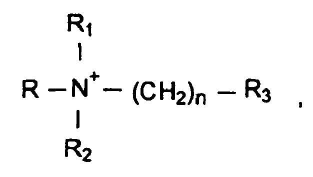 wobei R, R₁, R₂, R₃ und n die obengenannte Bedeutung haben, sind bevorzugt.

Geeignet sind insbesondere auch Handelsprodukte wie beispielsweise "Tegobetaine^{®}", "Dehytone^{®}" wie "AB 30", "G" und "K", "Lonzaine^{®}", "Varion^{®}" wie "ADG" und "CAS", "Lexaine^{®}", "Chembetaine^{®}", "Mirataine^{®}", "Rewoteric^{®}", "Schercotaine^{®}", "Monteine LCQ^{®}"; ""Alkateric^{®}", "Amonyl^{®}", "Amphosol^{®}", "Cycloteric BET^{®}", "Emcol^{®}", "Empigen^{®}", "Mackam^{®}", "Monateric^{®}", "Unibetaine^{®}" und "Velvetex^{®}".

Die amphoteren bzw. zwitterionischen Tenside sind vorzugsweise in einer Menge von 0,25 bis 5 Gew.-%, insbesondere 0,5 bis 2,5 Gew.-%, berechnet auf die Gesamtzusammensetzung, enthalten.

Die erfindungsgemäßen Haarbehandlungsmittel können die in solchen wäßrigen Zubereitungen üblichen Stoffe enthalten.

Dies sind beispielsweise synthetische oder natürliche haarkonditionierende Polymere, vorzugsweise in einer Menge von 0,1 bis 2,5, insbesondere 0,25 bis 1,5 Gew.-% der Gesamtzusammensetzung.

Als geeignete kationische Polymere sind neben den altbekannten quaternären Cellulosederivaten des Typs "Polymer JR" insbesondere quaternisierte Homo- und Copolymere des Dimethyldiallylammoniumchlorids, wie sie unter dem Handelsnamen "Merquat" im Handel sind, quaternäre Vinylpyrrolidon-Copolymere, insbesondere mit Dialkylaminoalkyl(meth) acrylaten, wie sie unter dem Namen "Gafquat" bekannt sind, Copolymerisate aus Vinylpyrrolidon und Vinylimidazoliniummethochlorid, die unter dem Handelsnamen "Luviquat" angeboten werden, Polyamino-Polyamid-Derivate, beispielsweise Copolymere von Adipinsäure-Dimethylaminohydroxypropyldiethylentriamin, wie sie unter dem Namen "Cartaretine F" vertrieben werden, sowie auch bisquaternäre langkettige Ammoniumverbindungen der in der US-PS 4 157 388 beschriebenen Harnstoff-Struktur, die unter dem Handelsnamen "Mirapol A 15" im Handel sind, zu nennen.

Verwiesen wird in diesem Zusammenhang auch auf die in den DE-OSen 25 21 960, 28 11 010, 30 44 738 und 32 17 059 genannten kationaktiven Polymeren sowie die in der EP-A 337 354 auf den Seiten 3 bis 7 beschriebenen Produkte. Es können auch Mischungen verschiedener kationischer Polymerer eingesetzt werden.

Anstelle der kationischen Polymeren oder in Kombination mit denselben können auch nichtionische Polymere verwendet werden. Als geeignete nichtionische Polymere werden vor allem Vinylpyrrolidon-Homo- und Copolymerisate, insbesondere Polyvinylpyrrolidon selbst, Copolymere aus Vinylpyrrolidon und Vinylacetat oder Terpolymerisate aus Vinylpyrrolidon, Vinylacetat und Vinylpropionat, wie sie beispielsweise von der Firma BASF unter dem Handelsnamen "Luviskol" vertrieben werden, eingesetzt.

Es können jedoch auch (Co-)Polymerisate aus den verschiedenen Acryl- und Methacrylestern, Acrylamid und Methacrylamid, beispielsweise Polyacrylamid mit Molgewichten von über 100.000, Dimethylhydantoin-Formaldehyd-Harze, etc., verwendet werden. Selbstverständlich sind auch Mischungen aus verschiedenen nichtionischen Polymeren verwendbar.

Geeignet sind schließlich auch noch amphotere Polymere, z. B. die unter der Bezeichnung "Amphomer" vertriebenen Copolymerisate aus N-Octylacrylamid, N-Butylaminoethylmethacrylat und Acrylsäure, sowie die bekannten anionischen Produkte, die im Stand der Technik hinreichend beschrieben sind.

Die erfindungsgemäßen Haarbehandlungsmittel können, wenn sie als Emulsionen, Dispersionen, Lösungen, Gele oder Aerosole als Nachbehandlungsmittel eingesetzt werden, die in solchen Zusammensetzungen üblichen Zusätze enthalten, deren Art und Charakter von der Applikationsform des Mittels abhängig ist. Es sind dies Fette, Fettalkohole, Emulgatoren, pH-Regulatoren, kationische und anionische Farbstoffe, UV-Absorber, Lösungs- und Verdünnungsmittel, Lösungsvermittler, Konservierungsmittel, Parfums, etc.

Geeignete Fette und Öle, zu denen auch Wachse zählen, sind insbesondere natürliche Öle wie Avocadoöl, Cocosöl, Palmöl, Sesamöl, Erdnußöl, Spermöl, Sonnenblumenöl, Mandelöl, Pfirsichkemöl, Weizenkeimöl, Macadamianußöl, Nachtkerzenöl, Jojobaöl, Ricinusöl, oder auch Oliven- bzw. Sojaöl, Lanolin und dessen Derivate, ebenso Mineralöle wie Paraffinöl und Vaseline.

Synthetische Öle und Wachse sind beispielsweise Silikonöle, Polyethylenglykole, etc.

Weitere geeignete hydrophobe Komponenten sind insbesondere Fettalkohole, vorzugsweise solche mit etwa 8 bis 22 Kohlenstoffatomen im Molekül wie Myristyl-, Cetyl-, Stearylalkohol, Wachsalkohole und Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Polyethylenglykol- und Polyglycerylfettsäureester wie PEG-7-glycerylcocoat, Cetylpalmitat, etc.

Diese hydrophoben Komponenten sind in den erfindungsgemäßen Zusammensetzungen vorzugsweise in einer Gesamtmenge von etwa 0,5 bis etwa 10, insbesondere etwa 1 bis 7,5, vor allem etwa 1,5 bis 5 Gew.-%, berechnet auf die Gesamtzusammensetzung, enthalten.

Eine bevorzugte Gruppe von aktiven Ingredientien sind C₁₀-C₂₄-Fettsäuren, insbesondere Behensäure und Stearinsäure, vorzugsweise zwischen 0,1 und 10 Gew.-% der Gesamtzusammensetzung.

Eine Zusammenfassung der Herstellung solcher Mittel findet sich in der bereits erwähnten Monographie von K. Schrader, S. 798 bis 815, insbesondere S. 804 ff.

Die erfindungsgemäßen Haarbehandlungmittel liegen als Emulsion, Dispersion oder (gegebenenfalls verdickte, d. h. als Gel) Lösung vor und können auch als Aerosolschaum konfektioniert werden. Diese Zusammensetzungen und ihre Herstellung sind dem Fachmann grundsätzlich bekannt und bedürfen daher keiner näheren Erläuterung.

Der pH-Wert der erfindungsgemäßen Haarbehandlungsmittel liegt vorzugsweise bei 3 bis 8, insbesondere zwischen 4 und 6.

Die folgenden Beispiele beschreiben Zusammensetzungen der erfindungsgemäßen Mittel. Es wurden Nachbehandlungsmittel der folgenden Zusammensetzungen durch Vermischen der Bestandteile, gegebenenfalls in Form von Vormischungen, hergestellt.

### Beispiel 1

| **Haarspülung** | |
|---|---|
| Cetylstearylalkohol | 1,00 (Gew.-%) |
| Mandelöl | 0,50 |
| PEG-7 Glycerylcocoat | 0,50 |
| Hydroxyethylcellulose | 1,00 |
| Saccharose | 0,50 |
| Benzophenone-4 | 0,30 |
| Dimethicone Copolyol Beeswax | 0,80 |
| Verbindung der Formel I* | 1,00 |
| Decylglucosid | 0,50 |
| 1,2-Propylenglykol | 1,00 |
| Dimethicone | 0,20 |
| Behentrimoniumchlorid | 0,40 |
| parfum | 0,30 |
| Konservierungsmittel (Parabene) | 0,20 |
| Farbstoff | 0,20 |
| Wasser | ad 100,00 |

| | |
|---|---|
| *R¹=R²=Oleyl; R³=CH₃;R⁴=CH₂CH₂OH;x=y=0; Y⁻=CH₃S0⁻₄. | |

### Vergleichstest

Eine Zusammensetzung 1 nach Beispiel 1 wurde an 10 Probanden im Halbseitentest auf eine Hälfte des Haares aufgebracht und im Vergleich zur anderen Haarhälfte, die mit einer ansonsten identischen Zusammensetzung 1 A, die anstelle der Saccharose den gleichen Anteil Behentrimethylammoniumchlorid enthielt, behandelt worden war, die folgenden Parameter nach einer einfachen Besser-gleich-schlechter Bewertung von 2 Friseuren manuell und visuell bestimmt:

| **Ergebnis Präferenz** | **Zusammensetzung 1** | **Gleich** | **Zusammensetzung 1A** |
|---|---|---|---|
| Naßkämmbarkeit | 16 | 4 | 0 |
| Naßgriff | 12 | 8 | 0 |
| Trockenkämmbarkeit | 16 | 4 | 0 |
| Trockengriff | 15 | 4 | 1 |
| Fülle (Volumen, Body) | 16 | 3 | 1 |
| Glanz | 14 | 5 | 1 |

Daraus ergibt sich eindeutig die überlegene haarkonditionierende Wirksamkeit der erfindungsgemäßen Zusammensetzung.

### Beispiel 2

| **Leave-on-Konditionier-Sprüh-Lotion** | |
|---|---|
| Saccharose | 0,3 (Gew.-%) |
| Fruktose | 0,3 |
| Verbindung der Formel I* | 0,8 |
| Amodimethicone | 0,5 |
| Panthenol | 0,5 |
| PEG-40 Hydriertes Ricinusöl | 0,3 |
| Polyquaternium-11 | 0,3 |
| Octylmethoxycinnamat | 0,1 |
| Talgtrimoniumchlorid | 0,1 |
| Milchsäure | 0,2 |
| Seidenproteinhydrolysat | 0,1 |
| Parfum | 0,2 |
| Ethanol | 42,0 |
| Wasser | ad 100,0 |

| | |
|---|---|
| *R¹=R²=Stearyl; R³=CH₃; R⁴=CH₂-CH₂-OH; x=y=0; Y=Cl⁻. | |

Das Produkt wird mittels einer Pumpsprühvorrichtung auf das Haar aufgebracht.

Nach dem Trocknen und Kämmen weist das Haar einen vollen Griff, guten Halt und dezenten Glanz auf.

### Beispiel 3

| **Haarpflege-Emulsion** | |
|---|---|
| Cetylstearylalkohol | 3,0 (Gew.-%) |
| Cetylalkohol | 1,0 |
| Octyldodecanol | 1,0 |
| Cetyltrimethylammoniumchlorid | 1,0 |
| Verbindung der Formel I* | 1,0 |
| Saccharose (als Honig) | 0,8 |
| Glyoxylsäure | 0,5 |
| Phosphorsäure | 0,2 |
| Cocobetain | 0,3 |
| Ammoniumhydroxid | 0,1 |
| Pflanzenextrakte | 0,3 |
| Konservierungsmittel | 0,1 |
| Parfum | 0,2 |
| Wasser | ad 100,0 |

| | |
|---|---|
| *R¹=R²=Oleyl; R³=CH₃; R⁴=CH₂-CH₂-OH; x=y=0; Y=CH₃S0₄⁻. | |

### Beispiel 4

| **Haarspülung** | |
|---|---|
| 1,2-Propylenglykol | 2,0 (Gew.-%) |
| Decylpolyglucosid | 1,5 |
| Saccharose | 0,5 |
| Verbindung der Formel I* | 1,0 |
| Dimethicone Copolyol Bienenwachs | 1,0 |
| Cetylstearylalkohol | 1,0 |
| Hydroxyethylcellulose | 0,8 |
| Kamillenextrakt | 0,3 |
| Benzophenone-4 | 0,2 |
| Behentrimoniumchlorid | 0,5 |
| Parfum | 0,2 |
| Methylparaben | 0,2 |
| Pyrrolidoncarbonsäure | 0,1 |
| Citronensäure | 0,1 |
| Wasser | ad 100,0 |

| | |
|---|---|
| *R¹=R²=Oleyl; R³=CH₃; R⁴=CH₂CH₂OH; x=y=0; Y⁻=CH₃SO₄⁻ | |

Die Zusammensetzung weist auf dem Haar ausgezeichnete Konditioniereigenschaften auf.

### Beispiel 5

| **Farbglanzspülung** | |
|---|---|
| Saccharose | 0,30 (Gew.-%) |
| Verbindung der Formel I* | 1,00 |
| Decylglucosid | 0,30 |
| Cetylstearylalkohol | 1,00 |
| 1,2Propandiol | 1,00 |
| Hydroxyethylcellulose | 1,00 |
| Dimethicone Copolyol Beeswax | 0,70 |
| Behenyltrimoniumchlorid | 0,40 |
| PEG-7 Glycerylcocoate | 0,50 |
| Methylparabene | 0,20 |
| Hennaextrakt | 0,05 |
| Basic Brown 17 | 0,03 |
| Basic Red 2 | 0,02 |
| Parfum | q.s. |
| Wasser | ad 100,00 |

| | |
|---|---|
| *R¹=R²=Oleyl; R³=CH₃; R⁴=CH₂CH₂OH; x=y=0; Y⁻=CH₅SO₄⁻ | |

Bei Anwendung dieser Spülung wurde nicht nur ein guter Halt und voller Griff der Frisur, sondern auch eine glänzende, dauerhafte, intensive Rotfärbung der Haare erhalten.

## Patentansprüche

1. Haarbehandlungsmittel auf wäßriger Grundlage, enthaltend ein Gemisch aus
a) 0,1 bis 20 Gew.-% mindestens einer Verbindung der allgemeinen Formel I 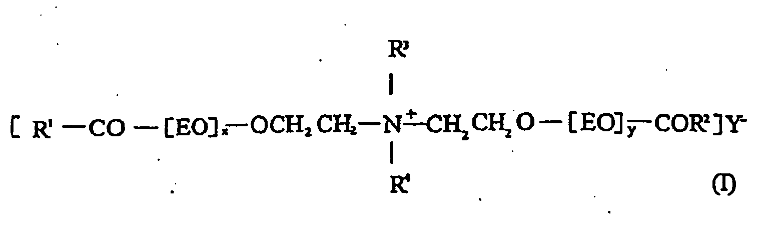 in der R¹ und R² jeweils für eine gegebenenfalls hydroxysubstituierte C₈-C₂₂- Alkyl- oder Alkenylgruppe, R³ und R⁴ für eine C₁-C₃-Alkylgruppe oder eine Gruppe CH₂-CH₂-O-[EO]_{z}-H, sowie x,y und z für 0 bis 5, und Y⁻ für ein Anion stehen, und
b) 0,1 bis 20 Gew.-% mindestens eines Mono- und/oder Oligosaccharids,
jeweils berechnet auf die Gesamtzusammensetzung des Mittels, wobei das Mittel ein Mono- und/oder Oligosaccharid ausgewählt aus Saccharose, Fructose, Lactose, Maltose, Galactose, Cellobiose, Fucose, Mannose, Erythrose und Rhamnose enthält.

2. Haarbehandlungsmittel nach Anspruch 1, enthaltend 0,5 bis 10 Gew.-% mindestens einer Verbindung der Formel I, berechnet auf die Gesamtzusammensetzung.

3. Haarbehandlungsmittel nach einem der Ansprüche 1 bis 2, **dadurch gekennzeichnet, daß** die Reste R¹ und R² jeweils einen Oleylrest, der Rest R³ eine Methylgruppe, der Rest R⁴ eine Gruppe -CH₂-CH₂-OH, und x und y 0 bedeuten.

4. Haarbehandlungsmittel nach einem der Ansprüche 1 bis 2, **dadurch gekennzeichnet, daß** die Reste R¹ und R² jeweils eine C₁₂-C₁₈-Alkylgruppe, der Rest R³ eine Methylgruppe, und der Rest R⁴ eine Gruppe -CH₂-CH₂-O-[EO]_{z}H, und x, y und z 0 bedeuten.

5. Haarbehandlungsmitter nach einem oder mehreren der Ansprüche 1 bis 4, enthaltend 0,25 bis 10 Gew.-%, berechnet auf die Gesamtzusammensetzung, mindestens eines Mono-und/oder Oligosaccharids ausgewählt aus Saccharose, Fructose, Lactose, Maltose, Galactose, Cellobiose, Fucose, Mannose, Erythrose und Rhamnose.

6. Haarbehandlungsmittel nach einem oder mehreren der Ansprüche 1 bis 5, enthaltend 0,5 bis 10 Gew.-% mindestens eines C₁₀-C₂₄-Fettalkohols, berechnet auf die Gesamtzusammensetzung.

7. Haarbehandlungsmittel nach einem oder mehreren der Ansprüche 1 bis 6, enthaltend mindestens einen anionischen und/oder kationischen Farbstoff.

## Claims

1. Hair treatment composition on aqueous basis, comprising a mixture of
a) 0.1 % to 20% by weight of at least one compound of the general formula I 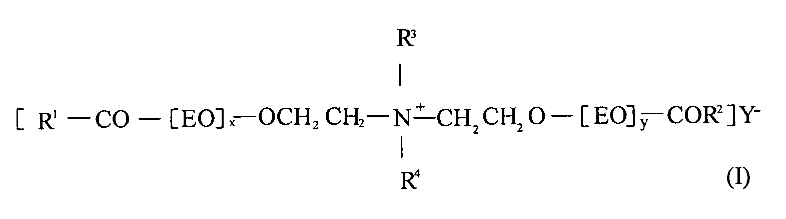 wherein R¹ and R² each stand for an optionally hydroxy substituted C₈-C₂₂-alkyl or alkenyl group, R³ and R⁴ stand for a C₁-C₃-alkyl group or a group -CH₂-CH₂-O-[EO]_{z}-H, and x, y and z stand for 0 to 5, and Y⁻ stands for an anion,
and
b) 0.1 % to 20% by weight of at least one mono- and/or oligosaccharide,
each calculated to the total composition whereby the composition comprises one mono- and/or oligosaccharide, selected from saccharose, fructose, lactose, maltose, galactose, cellobiose, fucose, mannose, erythrose, rhamnose,.

2. Hair treatment composition according to claim 1, comprising 0.5% to 10% by weight of at least one compound of the formula I, calculated to the total composition.

3. Hair treatment composition according to one of claims 1 to 2, wherein the groups R¹ and R² each stand for an oleyl group, the group R³ stands for a methyl group, the group R⁴ stands for a group -CH₂-CH₂-OH, and x and y are 0.

4. Hair treatment composition according to one of claims 1 to 2, wherein the groups R¹ and R² each stand for a C₁₂-C₁₈-alkyl group, the group R³ stands for a methyl group, and the group R⁴ stands for a group -CH₂-CH₂-O-[EO]_{z}H, and x, y and z are 0.

5. Hair treatment composition according to one or more of claims 1 to 4, comprising 0.25% to 10% by weight, calculated to the total composition, of at least one mono- and/or oligo- saccharide selected from saccharose, fructose, lactose, maltose, galactose, cellobiose, fucose, mannose, erythrose, rhamnose.

6. Hair treatment composition according to one or more of claims 1 to 5, comprising 0.5% to 10% by weight of at least one C₁₀-C₂₄-fafty alcohol, calculated to the total composition.

7. Hair treatment composition according to one or more of claims 1 to 6, comprising at least one anionic or cationic dyestuff.

## Revendications

1. Agent de traitement des cheveux à base aqueuse, qui contient un mélange constitué de :
a) 0.1 % à 20% en poids d'au moins un composé de formule génerale I 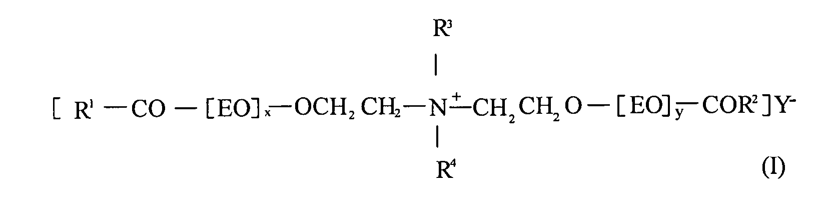 dans laquelle R¹ and R² représentent chacun un groupe alkyle ou alcényle en C₈-C₂₂, éventuellement hydroxylé, R³ et R⁴ représentent un groupe alkyle C₁ à C₃ ou un groupe -CH₂-CH₂-O-[EO]_{z}-H, x, y et z représentant un nombre de 0 à 5 et Y⁻ un anion,
et de
b) 0.1 % à 20% en poids d'au moins un mono- et/ou oligosaccharide,
chaque fois par rapport à la composition totale de l'agent ;composition de l'agent comprenant un mono- et/ou oligosaccharide, sélectionné parmi le saccharose, le fructose, le lactose, le maltose, le galactose, le cellobiose, le fucose, le mannose, l'érythrose et le rhamnose.

2. Agent de traitement des cheveux selon la revendication 1, qui contient de 0,5 à 10% en poids d'au moins un composé de formule I par rapport à la composition totale.

3. Agent de traitement des cheveux selon l'une des revendications 1 à 2, **caractérisé en ce que** les groupes R¹ et R² représentent chacun un groupe oléyle, le group R³ un groupe méthyle, le group R⁴ un groupe -CH₂-CH₂-OH, x et y représentant 0.

4. Agent de traitement des cheveux selon l'une des revendications 1 à 2, **caractérisé en ce que** les groupes R¹ et R² représentent chacun un groupe alkyle en C₁₂ à C₁₈, le groupe R³ représentant un groupe méthyle et le groupe R⁴ un groupe -CH₂-CH₂-O-[EO]_{z}H, x, y et z représentant 0.

5. Agent de traitement des cheveux selon l'une ou plusieurs des revendications 1 à 4, qui contient de 0,25 à 10% en poids par rapport à la composition totale d'au moins un mono- et/ou oligosaccharide sélectionné parmi le saccharose, le fructose, le lactose, le maltose, le galactose, le cellobiose, le fucose, le mannose, l'érythrose et le rhamnose.

6. Agent de traitement des cheveux selon l'une ou plusieurs des revendications 1 à 5, qui contient de 0,5 à 10% en poids d'au moins un alcool gras en C₁₀ à C₂₄ par rapport à la composition totale.

7. Agent de traitement des cheveux selon l'une ou plusieurs des revendications 1 à 6, qui contient au moins un colorant anionique et/ou cationique.
